# EUROPEAN PATENT APPLICATION

(11) **EP 3 623 035 A1**
(43) Date of publication of application: **18.03.2020**
(21) Application number: 19179970.9
(22) Date of filing: 13.06.2019
(51) Int. Cl.: B01D 53/14, B01D 53/30, B01D 53/34, B01D 53/77

(54) **GAS PURIFYING APPARATUS**

(30) Priority: 13.09.2018 US 201862730551 P; 30.10.2018 TW 107138399
(71) Applicant: Su, Chih-Hui, Taoyuan City 337 (TW); Li, Tien-Hua, Yilan County 270 (TW); Chang, Chih-Hsiung, Tapei City 115 (TW); Wang, Sheng-Wen, New Taipei City 244 (TW); Tsai, Ming-Chin, Keelung City 203 (TW)
(72) Inventor: Su, Chih-Hui, Taoyuan City 337 (TW); Li, Tien-Hua, Yilan County 270 (TW); Chang, Chih-Hsiung, Tapei City 115 (TW); Wang, Sheng-Wen, New Taipei City 244 (TW); Tsai, Ming-Chin, Keelung City 203 (TW)
(74) Representative: Becker & Kurig Partnerschaft Patentanwälte PartmbB

(57) **Abstract**

A gas purifying apparatus is used to receive chemical liquid mixing with a gas, and to clean the gas. The gas purifying apparatus includes a plurality of liquid status detection sensors, a plurality of gas status detection sensors, a plurality of pumping motors, a gas driving motor and a controller. The liquid status detection sensors are disposed in a chemical liquid transmission path to detect a plurality of liquid status information of the chemical liquid. The gas status detection sensors are disposed in a gas transmission path to detect a plurality of gas status information of the gas. The controller performs an operation on the liquid status information and the gas status information to adjust a first setting value and a second setting value. The first setting value and the second setting value are respectively used to drive the pumping motors and the gas driving motor.

## Description

### BACKGROUND

### Field of the Disclosure

The disclosure relates to a gas purifying apparatus, and more particularly to an intelligent gas purifying apparatus.

### Description of Related Art

In the field of the prior art, a gas purifying apparatus focuses on detection of physical feature signals, and determines whether the gas purifying apparatus has an abnormal operation occurred according to the detected physical feature signals, and performs operation of shutdown and maintenance. That is to say, in the prior art, only the operation of the gas purifying apparatus can be monitored, and online adjustment actions cannot be performed immediately for the gas purifying apparatus, and the working efficiency of the gas purifying apparatus can't be maintained.

### SUMMARY

The disclosure provides a gas purifying apparatus, which can adjust a setting value of a pump motor and a gas driving motor in real-time to improve the working efficiency.

The gas purifying apparatus of the disclosure is used to receive chemical liquid to mix with a gas and to purify the gas. The gas purifying apparatus includes a plurality of liquid status detection sensors, a plurality of gas status detection sensors, a plurality of pumping motors, a gas driving motor, and a controller. The plurality of liquid status detection sensors are disposed in a transmission path of the chemical liquid, configured to detect a plurality of liquid status information of the chemical liquid. The gas status detection sensors are disposed in a transmission path of gas, configured to detect multiple gas status information of the gas. The controller is coupled to the gas status detection sensors and the liquid status detection sensors, and performs calculations on the gas status information and the gas status information to adjust a first setting value and a second setting value.

In an embodiment of the disclosure, the gas purifying apparatus further includes a plurality of vibration detectors and a plurality of electrical signal detectors. The vibration detector is disposed adjacent to the pump motor and the gas driving motor respectively to detect multiple vibration information of the pump motor and the gas driving motor. The electrical signal detectors are coupled to the pump motor and the gas driving motor to detect multiple driving signals of the pump motor and the gas driving motor.

In an embodiment of the disclosure, the controller further adjusts at least one of the first setting value and the second setting value according to the vibration information and the driving signal.

In an embodiment of the disclosure, the controller extracts a plurality of liquid status feature values of the liquid status information, extracts a plurality of gas status feature values of the gas status information, and the liquid status feature values and the gas status feature values are input to a neural network system to generate the first setting value and the second setting value.

In an embodiment of the disclosure, the controller performs a training operation on the neural network system according to the received liquid status feature value and the gas status feature value in the training time interval, and thereby adjusting multiple weight values in the neural network system.

In an embodiment of the disclosure, the liquid status detection sensors include a liquid pressure detector, a pH value detector, a redox potential detector, a liquid temperature detector, a liquid flow detector, and a conductivity detector.

In an embodiment of the disclosure, the gas status detection sensors include a gas temperature detector, a gas concentration detector, and an anemometer.

In an embodiment of the disclosure, the gas temperature detector and the gas concentration detector are respectively disposed at an air inlet and an air outlet of the gas purifying apparatus, and the anemometer is disposed at the air outlet of the gas purifying apparatus.

In an embodiment of the disclosure, the gas purifying apparatus further includes a display device. The display device is coupled to the controller to display at least one of the liquid status information, the gas status information, the first setting value, and the second setting value.

In an embodiment of the disclosure, the gas purifying apparatus further includes a dosing tank, a dosing motor, and a dosing tank level detector. The dosing liquid tank is used to hold chemical drugs. The dosing motor is coupled to the dosing liquid tank to provide a chemical to mix with water to produce chemical liquid. The dosing tank liquid level detector is coupled to the dosing liquid tank to detect the liquid level of the dosing liquid tank on the dosing liquid tank.

Based on the above, the gas purifying apparatus of the disclosure disposes a controller, and configures the controller to perform calculation on the liquid status information and the gas status information to adjust the first setting value and the second setting value for controlling the pump motor and the gas driving motor. In this way, the operation of the pump motor and the gas driving motor of the gas purifying apparatus can be adjusted according to the actual physical status of the gas and liquid in the gas purifying apparatus, and the working efficiency of the gas purifying apparatus can be maintained and improved.

The above described features and advantages of the disclosure will be more apparent from the following description.

### BRIEF DESCRIPTION OF THE DRAWINGS

FIG. 1 is a schematic view of a gas purifying apparatus in accordance with an embodiment of the disclosure.
FIG. 2 is a schematic diagram illustrating an operation flow of a controller according to an embodiment of the disclosure.
FIG. 3 is a block diagram of a neural network system according to an embodiment of the disclosure.
FIG. 4 is a schematic diagram illustrating an operation of a neural network learning method according to an embodiment of the disclosure.
FIG. 5 is a schematic view of a gas purifying apparatus according to another embodiment of the disclosure.

### DESCRIPTION OF THE EMBODIMENTS

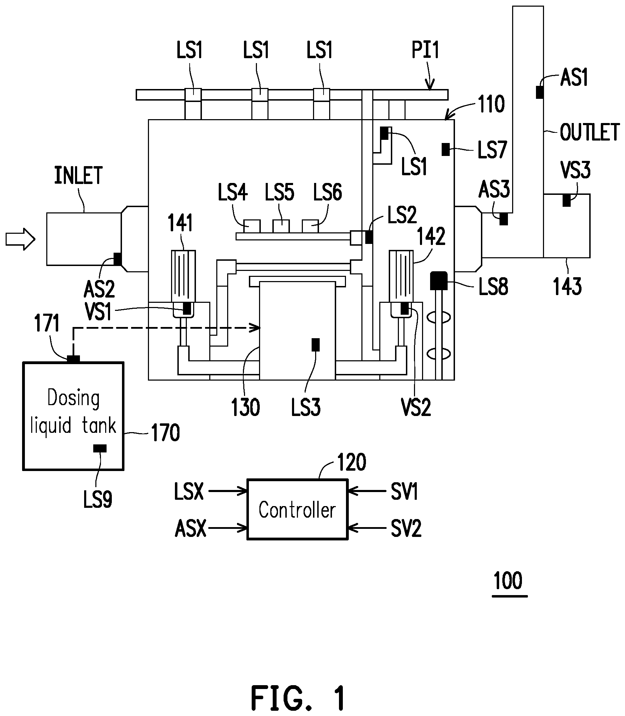

FIG. 1 is a schematic view of a gas purifying apparatus in accordance with an embodiment of the disclosure. The gas purifying apparatus 100 has a shell 110, the gas purifying apparatus 100 further includes a plurality of liquid status detection sensors LS1∼LS8, a plurality of gas status detection sensors AS1∼AS3, pump motors 141, 142, a gas driving motor 143, and a controller 120. In the embodiment, the liquid status detection sensors LS1 ∼ LS8 are disposed at a plurality of different locations in a transport channel PI1 for transporting the chemical liquid. The liquid status detection sensors LS1-LS8 detect a plurality of liquid status information LSX of the chemical liquid in a transmission path PI1. The gas status detection sensors AS1-AS3 are disposed at a plurality of different locations in a transmission path for transporting gas, configured to detect a plurality of gas status information ASX of the gas. Wherein, the transmission path of the transport gas may be formed between a gas inlet INLET and a gas outlet OUTLET of the gas purifying apparatus 100. The chemical liquid can be stored in a chemical liquid tank 130. In the embodiment, the chemical liquid tank 130 is coupled to the dosing liquid tank 170. A dosing motor 171 is disposed on the dosing liquid tank 170, and is used to inject chemical material into the chemical liquid tank 130 to mix the chemical material with water to produce the chemical liquid. A type of the chemical liquid can be configured depending on the soiled material in the gas in which it is placed, with no fixed restrictions.

The controller 120 is coupled to the liquid status detection sensors LS1-LS9 and the gas status detection sensors AS1-AS3. The controller 120 receives the liquid status information LSX and the gas status information ASX generated by the liquid status detection sensors LS1-LS9 and the gas status detection sensors AS1-AS3 respectively. The controller 120 performs operations on the liquid status information LSX and the gas status information ASX, and thereby adjusts setting values SV1 and SV2. Herein, the value SV1 is set to be supplied to the pump motor 141, 142, and the pump motors 141, 142 adjust a driving pressure supplied to the chemical liquid according to the setting value. The setting value SV2 is provided to a gas driving motor 143 (for example, a fan), and the gas driving motor 143 configures a flow rate of the gas according to the setting value SV2.

In terms of implementation details, when performing a gas purifying operation, the gas may pass through a pretreatment apparatus of the gas purifying apparatus 100 (which may be disposed in the upper half of the shell 110), and through spraying the chemical liquid on the gas to perform a first cleaning process. Then, after the chemical liquid and the gas are sufficiently mixed, the mixed fluid can be subjected to a main cleaning process by the gas purifying apparatus 100 (which can be disposed in the lower half of the shell 110) to purify pollutants in the gas.

It is worth mentioning that the key factors affecting the efficiency of the gas purifying operation includes a system water pressure of the chemical liquid, a material to be treated, and a pH value. Therefore, in the embodiment of the disclosure, the liquid status detection sensor LS1 may be a liquid pressure detector; the liquid status detection sensor LS2 may be a liquid flow detector; the liquid status detection sensor LS3 may be a liquid temperature detector; the liquid status detection sensors LS4 may be a redox potential detector; the liquid status detection sensors LS5 may be a pH detector; and the liquid status detection sensors LS6 may be a conductivity detector. In addition, in the embodiment, the liquid status detection sensors LS7 is provided as a differential pressure detector; the liquid status detection sensors LS8 as the dosing tank level detector and the liquid status detection sensors LS9 as another dosing tank level detector.

Then, a condensed action of the treated gas through the gas purifying apparatus 100 allows the gas concentration of the gas to be optimally purified and discharged to the atmosphere. In the embodiment of the disclosure, the gas status detection sensors AS2 may be a gas temperature detector; the gas status detection sensors AS3 may be a gas concentration detector; and the gas status detection sensors AS1 may be an anemometer. Moreover, the gas status detection sensor AS2 and the gas status detection sensor AS3 are respectively disposed at the air inlet INLET and the air outlet OUTLET of the gas purifying apparatus 100, and the gas status detection sensor AS1 is disposed at the air outlet OUTLET of the gas purifying apparatus 100.

In the embodiment, the controller 120 can perform calculations according to the adaptive learning model algorithm (for example, a neural network system) for the liquid status detection sensors LS1 to LS8 and the gas status detection sensors AS1 to AS3, and adjust the setting values SV1, SV2 accordingly. Herein, a setting value SV1 can be set correspond to the driving current of the pump motors 141 and 142, and a setting value SV2 can be set correspond to the driving current of the gas driving motor 143. By adjusting the size of the setting value SV1, it is possible to control the rotational speed, the output and other related features of at least one of the pumping motors 141 and 142. By adjusting the size of the setting value SV2, it is possible to control the rotational speed, the output and other related features of the gas driving motor 143.

Incidentally, in order to monitor the operating statuses of the pump motor 141, 142 and the gas driving motor 143, the gas purifying apparatus 100 of the embodiment of the disclosure is adjacent to the pump motors 141, 142 and the gas driving motor 143 to respectively dispose a plurality of vibration detectors VS1, VS2, and VS3. The vibration detectors VS1, VS2, and VS3 detect the vibration statuses of the pump motors 141, 142 and the gas driving motor 143, respectively, and generate a plurality of vibration information. In addition, the gas purifying apparatus 100 of the embodiment of disclosure may be provided with an electrical signal detector to detect a plurality of driving signals (driving voltage and/or driving current) of the pump motors 141, 142 and the gas driving motor 143, and working statuses of the pump motors 141, 142 and the gas driving motor 143 are thus known.

On the other hand, the dosing motor 171 can control an amount of chemical injection according to the pH value of the chemical liquid detected by the liquid status detection sensor LS5 (pH level detector), and along with the water pressure of the chemical liquid and the type of gas being treated detected by the liquid status detection sensor LS9 (dosing liquid level detector) and the liquid status detection sensor LS1 (liquid pressure detector). The control mechanism of the dosing motor 171 is configured to mix spraying chemical with water and then mix the chemical liquid with the gas, thereby improving the processing efficiency. Furthermore, the overall efficiency of the gas purifying apparatus 100 can be improved by combining the information of the water pressure of the chemical liquid, and the control of the amount of wind and the wind pressure generated by the gas driving motor.

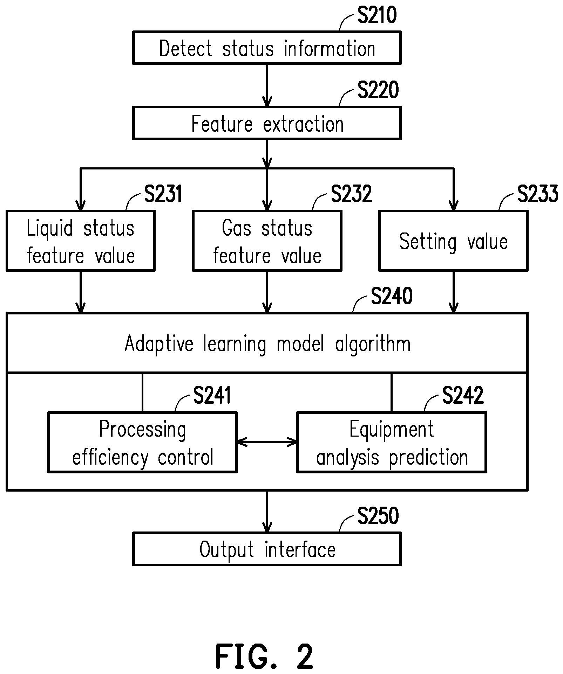

Please refer to FIG. 2 below. FIG. 2 is a schematic diagram illustrating the operation flow of the controller according to an embodiment of the disclosure. In FIG. 2, the controller drives the liquid status detection sensors and the gas status detection sensors to detect a plurality of liquid status information and gas status information in step S210. Step S220 performs a feature extraction operation on the liquid status information and the gas status information, and generates a liquid status feature value (step S231) and a gas status feature value (step S232), respectively. In addition, the controller provides a current setting value (step S233) corresponding to the pump motor and the gas driving motor, and then performs the adaptive learning model algorithm through step S240 to generate an adjusted setting value, and output the adjusted setting value by the output interface (step S250).

In step S240, the controller may perform calculations for a processing efficiency control (step S241) and an equipment analysis prediction (step S242) of the gas purifying apparatus, and generate a corresponding setting value, in addition to monitoring the equipment status, the working performance of the gas purifying apparatus can be improved through the adjusted setting value. Moreover, the adaptive learning model algorithm can be a type of neural network system.

Regarding the details of the feature extraction step (step S220), the controller of the embodiment of the disclosure may perform a fast Fourier transform and a normalized averaging method to retain effective information and filter out invalid noise information.

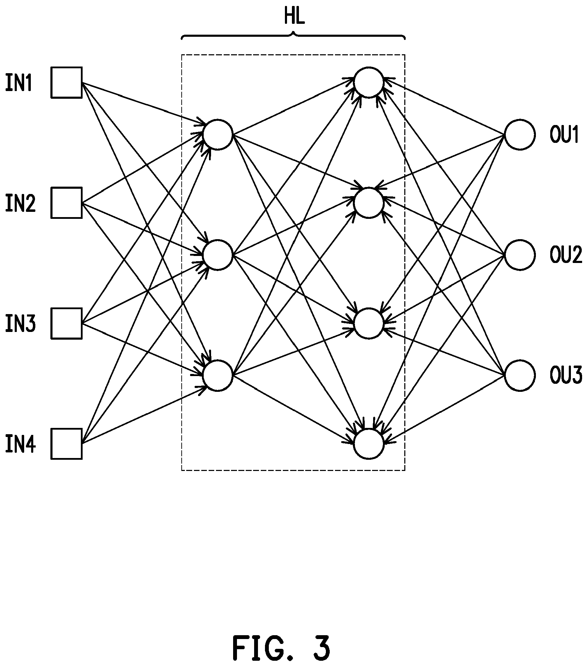

Please refer to FIG. 3 below. FIG. 3 is a structural diagram of a neural network system according to an embodiment of the disclosure. The neural network system can have multiple levels, including an input layer, multiple hidden layers HLs, and an output layer. Among them, the input layer receives multiple inputs IN1-IN4 as feature values, and through multiple hidden layers HLs, multiple setting values OU1-OU3 are generated in the output layer. In the embodiment, the setting values OU1 to OU3 are configured to control the actions of the pump motor and the gas driving motor.

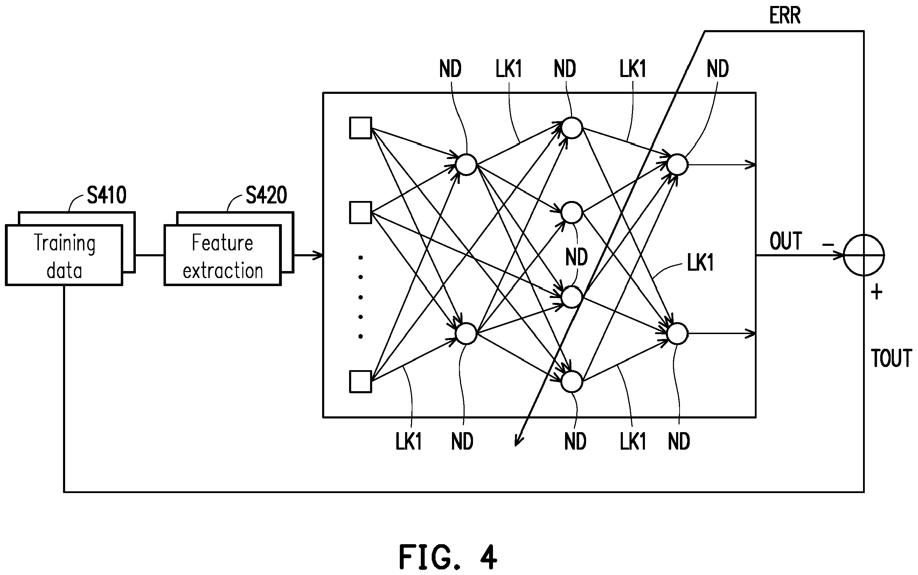

Referring to FIG. 4, FIG. 4 is a schematic diagram illustrating an operation of a neural network system learning model according to an embodiment of the disclosure. In the gas purifying apparatus of the embodiment of the disclosure, the controller receives a plurality of training materials (including the gas status information and the liquid status information) within a training time interval in step S410, among them, the training data has a corresponding expected output TOUT. Step S420 performs feature extraction on the training data, and inputs the extracted feature values into the neural network system. The neural network system includes a plurality of nodes ND, and the adjacent two nodes may have corresponding one or more linking node LK1, and the linking node LK1 has a plurality of weight values respectively. Error information ERR can be generated by subtracting the output OUT (e.g., setting value) generated by the neural network system and the expected output TOUT. The error information ERR can be fed back to the neural network system and used as the basis for adjusting the weight values.

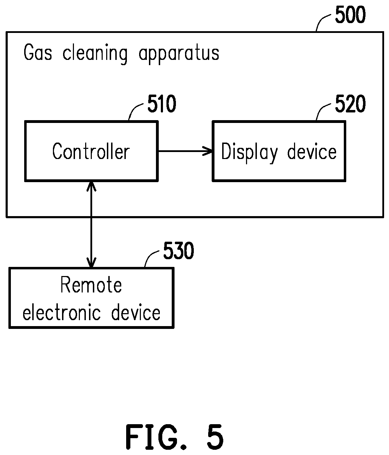

Please refer to FIG. 5. FIG. 5 is a schematic diagram of a gas purifying apparatus according to another embodiment of the disclosure. The gas purifying apparatus 500 includes a controller 510 and a display device 520. The controller 510 and the display device 520 are coupled to each other. The display device 520 can be used to display at least one of the liquid status information, gas status information, a first setting value, and a second setting value. The display device 520 can also be used to generate a warning signal, which generates the warning signal when a working state of equipment is unstable or a fault occurs in the gas purifying apparatus.

In the embodiment, the gas purifying apparatus 500 can be connected to a remote electronic device 530 through the controller 510, and at least one of the liquid information, the gas status information, the first setting value, and the second setting value is transmitted to the remote electronic device 530. In this way, the operator of the gas purifying apparatus 500 can perform the monitoring operation of the gas purifying apparatus 500 through the remote electronic device 530, thereby improving the safety of the gas purifying apparatus 500.

In summary, the gas purifying apparatus of the embodiment of the disclosure detects a plurality of statuses of the chemical liquid and the gas therein, and performs operations according to the detected plurality of liquid status signals and gas status signals, and generates setting values of the motor (pump motor and gas driving) used to drive the chemical liquid and gas. By adaptively adjusting output features of the pump motor and the gas driving motor to optimize the operation of the gas purifying apparatus, the efficiency of the gas purifying apparatus is improved.

## Claims

1. A gas purifying apparatus (100), configured for receiving chemical liquid to be mixed with gas to purify the gas, the gas purifying apparatus (100) comprising:
a plurality of liquid status detection sensors (LS1-LS8), disposed in a transmission path (PI1) for transmitting the chemical liquid, configured to detect a plurality of liquid status information (LSX) of the chemical liquid;
a plurality of gas status detection sensors (AS1-AS3), disposed in the transmission path (PI1) for transmitting the gas, configured to detect a plurality of gas status information (ASX) of the gas;
a plurality of pumping motors (141, 142), configured for providing a driving pressure of the chemical liquid according to a first setting value (SV1);
a gas driving motor (143), configured for setting a flow rate of the gas according to a second setting value (SV2);
a controller (120), coupled to the plurality of liquid status detection sensors (LS1-LS8) and the plurality of gas status detection sensors (AS1-AS3), and performing operations on the plurality of liquid status information (LSX) and the plurality of gas status information (ASX) to adjust the first setting value (SV1) and the second setting value (SV2).

2. The gas purifying apparatus (100) as claimed in claim 1 further comprises:
a plurality of vibration detectors (VS1-VS3), disposed adjacent to the plurality of pumping motors (141, 142) and the gas driving motor (143), respectively, configured for detecting a plurality of vibration information of the plurality of pumping motors (141, 142) and the gas driving motor (143); and
a plurality of electrical signal detectors, respectively coupled to the plurality of pumping motors (141, 142) and the gas driving motor (143) to detect a plurality of driving signals of the plurality of pump motor (141, 142) and the gas driving motor (143).

3. The gas purifying apparatus (100) as claimed in claim 2, wherein the controller (120) further adjusts at least one of the first setting value (SV1) and the second setting value (SV2) according to the plurality of vibration information (VS1-VS3) and the plurality of driving signals.

4. The gas purifying apparatus (100) as claimed in claim 1, wherein the controller (120) extracts a plurality of liquid status feature values of the plurality of liquid status information (LSX), extracts a plurality of gas status feature values of the plurality of gas status information (ASX), and inputs the plurality of liquid status feature values and the plurality of gas status feature values to a neural network system to generate the first setting value (SV1) and the second setting value (SV2).

5. The gas purifying apparatus (100) as claimed in claim 4, wherein the controller performs a training action on the neural network system according to the received plurality of liquid status feature values and the plurality of gas status feature values within a training time interval, so as to adjust a plurality of weight values in the neural network system.

6. The gas purifying apparatus (100) as claimed in claim 1, wherein the plurality of liquid status detection sensors (LS1-LS8) comprise a liquid pressure detector, a pH detector, a redox potential detector, a liquid flow detector, a liquid temperature detector, and a conductivity detector.

7. The gas purifying apparatus (100) as claimed in claim 1, wherein the gas status detection sensors comprise a gas temperature detector, a gas concentration detector, and an anemometer.

8. The gas purifying apparatus (100) as claimed in claim 7, wherein the gas temperature detector and the gas concentration detector are respectively disposed at an air inlet (INLET) and an air outlet (OUTLET) of the gas purifying apparatus, and the anemometer is disposed at the air outlet (OUTLET) of the gas purifying apparatus (100).

9. The gas purifying apparatus (100, 500) as claimed in claim 1 further comprises:
a display device (520), coupled to the controller (120, 510), configured for displaying at least one of the plurality of liquid gas status information (LSX), the plurality of gas status information (ASX), the first setting value (SV1), and the second setting value (SV2).

10. The gas purifying apparatus (100, 500) as claimed in claim 1, wherein the controller (120, 510) is further coupled to a remote electronic device (530), and provides to the remote electronic device (530) at least one of the plurality of liquid status information (LSX), the plurality of gas status information (ASX), the first setting value (SV1), and the second setting value (SV2).

11. The gas purifying apparatus (100) as claimed in claim 1 further comprises:
a dosing liquid tank (170), configured for containing chemical drugs;
a dosing motor (171), coupled to the dosing liquid tank to provide the chemical drugs to mix with water to produce chemical liquid; and
a dosing tank level detector (LS9), coupled to the dosing liquid tank (170) to detect the dosing tank level of the dosing liquid tank (170).
